# EUROPEAN PATENT APPLICATION

(11) **EP 4 684 770 A1**
(43) Date of publication of application: **28.01.2026**
(21) Application number: 25191509.6
(22) Date of filing: 24.07.2025
(51) Int. Cl.: A61F 9/008, G02F 1/35, G02F 1/355

(54) **OPHTHALMOLOGICAL OPTICAL DEVICE FOR MODIFYING A TREATMENT LASER BEAM AND AN OPHTHALMOLOGICAL LASER TREATMENT DEVICE COMPRISING THE OPHTHALMOLOGICAL OPTICAL DEVICE**

(30) Priority: 26.07.2024 CH 8032024
(71) Applicant: Ziemer Ophthalmic Systems AG, 2562 Port (CH)
(72) Inventor: RATHJEN, Christian, 28197 Bremen (DE); STEINLECHNER, Michael, 8006 Zürich (CH); STUDER, Thomas, 3286 Muntelier (CH)
(74) Representative: Rentsch Partner AG

(57) **Abstract**

The present disclosure relates to an ophthalmological optical device (1) for modifying at least one parameter (3) of a treatment laser beam (2) used for treating an eye of a patient and to an ophthalmological laser treatment device comprising the ophthalmological optical device (1), the ophthalmological optical device (1) comprising a housing (4), an optical component (11), which is arranged in an interior (5) of the housing (4), and which is configured to modify at least one parameter (3) of the treatment laser beam (2), a support assembly (13), which is arranged at least partially in the interior (5) of the housing (4) and which is configured to position the optical component (11) in the interior (5) of the housing (4) and a temperature control assembly (19), which is configured to control the temperature of the optical component (11).

## Description

### FIELD OF THE DISCLOSURE

The present disclosure relates to an ophthalmological optical device for modifying, adapting or amending at least one parameter of a treatment laser beam used for treating an eye of a patient and to an ophthalmological laser treatment device comprising the ophthalmological optical device.

### BACKGROUND OF THE DISCLOSURE

Ophthalmological laser treatment devices comprise a plurality of different components to create and project a desired treatment laser beam to the desired treatment position in an eye of a patient for treating the eye of the patient. It is highly important that the laser beam is projected at the right position at the eye of the patient. In order to achieve the desired positioning of the treatment laser beam, a plurality of devices, like cameras, adjustable mirrors and other optical devices control the trajectory of the treatment laser beam with respect to the monitored position of the eye of the patient during the treatment of the eye with the ophthalmological laser treatment devices. Further, it is highly important that the treatment laser beam has the desired properties in order to treat the eye of the patient, in particular the eye tissue, as desired. The parameters of the treatment laser beam may vary during the treatment. In case the parameters of the laser beam or of the pulsed laser beam are not within the desired ranges or limits, the eye of the patient could be harmed. It is therefore highly important to form the treatment laser beam with the desired parameters and to control the parameters along its trajectory from the laser source to the eye of the patient throughout the operation of the ophthalmological laser treatment device. Further, it may be required that the treatment laser beam has different properties for different treatments. Different eye tissue may require the usage of treatment laser beams having respective properties, which are specifically selected in dependence of the eye tissue to be treated and / or the type of treatment.

In order to control and / or to modify the treatment laser beam optical components, e.g. specific crystal elements or glass elements, are conventionally used, which are placed in the beam path of the treatment laser beam. The treatment laser beam interacts with the optical components, which could change at least one property or parameter of the treatment laser beam.

Small differences in the environment of the optical components, as the temperature or the atmospheric composition surrounding the specific crystal or glass element could affect the interaction of the treatment laser beam with the optical components such that the resulting parameters of the treatment laser beam deviate from the desired parameters.

### SUMMARY OF THE DISCLOSURE

It is therefore an object of the present disclosure to provide an ophthalmological optical device, which addresses at least some of the disadvantages of the known prior art and to provide an ophthalmological laser treatment device, which comprises such a device. In particular, it is an object of the present disclosure to provide an alternative and / or an improved ophthalmological optical device for modifying, adjusting and/or controlling at least one parameter of a treatment laser beam and an ophthalmological laser treatment device comprising such an ophthalmological optical device.

According to the present disclosure, these objects are addressed by the features of the independent claims. Moreover, further advantageous embodiments follow from the dependent claims and the description.

According to the present disclosure, an ophthalmological optical device for modifying at least one parameter of a treatment laser beam used for treating an eye of a patient is specified. The ophthalmological optical device comprises preferably a housing, an optical component, a support assembly and a temperature control assembly.

The housing is configured to provide in its interior an atmospheric environment, which differs from an atmospheric environment with respect to an exterior of the housing. In other words, the housing is configured to provide a space in its interior, which may differ with respect to atmospheric conditions, compared to the exterior, in particular the surrounding environment. The atmospheric conditions are for example, gas pressure, temperature and composition of the atmosphere, including the humidity, in the interior. Further differences are also conceivable. For example, the housing comprises housing walls, which separate the interior from the exterior, in particular in a hermetically or a gas tight manner. The housing may be a hermetically sealed housing. The interior of the housing may further be configured to provide and hold a negative pressure, in particular a vacuum, with respect to the exterior of the housing. Further, the housing might comprise at least partially an insulation layer, which is configured to provide insulation for the interior with respect to the exterior. In a further embodiment, the housing might be configured to received and hold a shielding gas or a protective gas in its interior. The housing may comprise a gas connection or gas interface, through which the shielding gas is insertable and removable. The housing is preferably designed to be gas-tight to hold the shielding gas and/or the negative pressure during the operation of the ophthalmological optical device.

The ophthalmological optical device further comprises an optical component, which is arranged in the interior of the housing, and which is configured to modify at least one parameter of the treatment laser beam when the treatment laser beam interacts with the optical component. The optical component is the component of the ophthalmological device, which is determined to modify or change the at least one of the parameters of the treatment laser beam as desired when the treatment laser beam interacts with the optical component. The optical component is e.g. made of a specific material, which modifies the parameter of the treatment laser beam. Interacting may comprise that the treatment laser beam penetrates the optical component, that the treatment laser beam is deflected by the optical component and/or that the treatment laser beam passes an area, which is influenced by the optical component such that the at least one parameter of the treatment laser beam is modified. Modifying may be determined in that the at least one parameter or the plurality of parameters differs after interacting with the optical component.

The parameter of the treatment laser beam, which could be modified by the optical component comprise for example the wavelength, the spectral distribution, polarization, pulse length, phase relationship between different wavelengths and within the wave front, Other parameters are also conceivable.

The ophthalmological optical device further comprises a support assembly, which is arranged partially or completely in the interior of the housing, and which is configured to position the optical component in the interior of the housing. In other words, the support assembly is the support for the optical component, which places and holds the optical component in the interior of the housing. The support assembly extends e.g. from at least one housing wall into the interior of the housing. The optical component may be arranged at the tip portion of the support assembly. The dimensions of the support assembly may determine the position of the optical component within the housing, in particular the distance of the optical component from the housing wall or walls. The support assembly may further be configured to move the optical component within the interior with respect to the housing. The support assembly might comprise a specific mechanical structure, which extends from at least one housing wall to the optical component. In another embodiment, the support assembly might comprise a rope construction, which holds the optical component at the desired position.

The ophthalmological optical device further comprises a temperature control assembly, which is configured to control the temperature of the optical component. The temperature of the optical component may affect the operation of the optical component. Optical materials may change their properties when the temperature of the optical materials change. In other words, the temperature of the optical component may determine the result or outcome of the interaction with the treatment laser beam. It is therefore of high importance to control the temperature of the optical component during the operation of the ophthalmological optical device. The ophthalmological optical device may be used in different environmental conditions e.g. different surrounding temperatures and humidity levels. Without a possibility to control the temperature of the optical component, it may not be possible to guarantee that the at least one parameter of the treatment laser beam is modified as desired. The temperature control assembly is therefore provided to control the temperature of the optical component and / or of the interior of the housing.

The ophthalmological optical device provides due to its components ideal conditions for modifying at least one parameter of the treatment laser beam as desired during the operation of the ophthalmological optical device. The atmospheric environment, the gas pressure surrounding the optical component and / or the temperature of the optical component is controlled such that the desired modification of the at least one parameter of the treatment laser beam is performed as desired. The ophthalmological optical device may form an optical device oven or optical crystal oven for ophthalmological applications.

The housing may comprise a sealing mechanism or is sealed such that the interior holds the atmospheric conditions.

In an embodiment, the optical component comprises an optical crystal or an optical glass, which is configured to modify at least one parameter of the treatment laser beam when the treatment laser beam interacts with the optical component. The optical crystal may be a non-linear crystal and/or a quartz crystal. Many different schemes and embodiments are known how non-linear crystals can be used to modify input laser beams e.g. by generating new wave-length out of the input beam. Further, it may be possible to combine two wavelengths to a new wavelength in the optical component. Non-linear crystals consist or are at least partially made of Lithium Niobate (LiNbO3) and Tantalate (LiTaO3), Potassium niobate (KNbO3), Potassium titanyl phosphate (KTP, KTiOPO4), KTA (KTiOAsO4), RTP (RbTiOPO4) and RTA (RbTiAsPO4), Potassium dihydrogen/dideuterium phosphate (KDP/KD*P), Borate Crystals, Mid-IR Crystals. Interacting may comprise that the treatment laser beam penetrates the optical crystal at one side and emerges from the crystal at the opposing side and / or that the treatment laser beam is deflected by the optical crystal. The specific optical crystals are in particular advantageous to modify the treatment laser beam, in particular because they provide the desired stable modification.

Some of these crystals are highly hygroscopic like KDP or CLBO (a Borate variant) which makes their use in laser systems not operated by laser specialist highly unreliable. BBO in particular profits from and operation in dry conditions where a temperature control in combination with alignment means for walk-off compensation enables robust operation at high efficiency.

In an embodiment, the optical component is arranged in a center area of the interior of the housing, which is spaced apart from each housing wall of the housing. The center area extends for example from the geometrical center of the housing to half of the distance from the geometrical center to each housing wall. The thereby determined area may be defined as center area. In a further embodiment, the center area extends for example from the geometrical center of the housing to two third of the distance from the geometrical center to each housing wall. Positioning the optical component within the center area ensures that it is spaced apart from each housing wall such that the housing walls and / or the environment has as little effect as possible or a uniform effect on the optical component. In particular, it is thereby possible to mitigate potential implications to the optical component from the housing wall, like heat transportation, radiation, etc.

In an embodiment, the support assembly comprises a mechanical structure, which extends from at least one of the housing walls to the optical component. The mechanical structure is e.g. a rod, a shaft or any other structure, which positions the optical component in the interior of the housing.

The support assembly may further comprise a moving mechanism, which is configured to move the optical component within the interior of the housing. As outlined above, the support assembly is configured to position the optical component at the desired position. The support assembly is according to this embodiment further configured to move the optical component, via the moving mechanism, within the interior of the housing. The support assembly thereby fulfills two purposes, namely positioning and moving. In order to achieve the desired modification of the treatment laser beam, it is of high importance that the optical component is placed in the beam path of the treatment laser beam as desired. Temperature differences or other implications might affect the position of the optical component with respect to the trajectory or beam path of the treatment laser beam. Repositioning the optical component could therefore be necessary to ensure the desired modification of the at least one parameter of the treatment laser beam. In an embodiment, the support assembly may comprise at least one sensor, which provides a sensor signal, which is indicative of the position of the optical component with respect to the housing and / or with respect to the trajectory of the treatment laser beam. The moving mechanism can be controlled using this sensor signal for repositioning of the optical component.

In a further embodiment, the moving mechanism may also be implemented in the rope construction, which forms the support structure as described in the embodiments above.

In an embodiment, the moving mechanism of the support assembly comprises at least one actuator or motor, preferably a plurality of actuators or motors, more preferably a plurality of actuators forming a moving structure e.g. a hexapod, which form at least partially the moving mechanism, and which is configured to move the optical component within the interior of the housing. The actuator, is e.g. a linear actuator, which is configured to perform a linear movement, thereby linearly moving the optical component with respect to the housing. Rotational actuators are also conceivable. By using a plurality of actuators, e.g. three it is possible to move the optical components in three dimensions. The specific arrangement of the actuators as moving structure e.g. the hexapod enables to move the optical component in particular freely. The hexapod is a special form of parallel kinematics machine that has six legs of variable length. The typical design of the hexapod enables movement in all six degrees of freedom (three translational and three rotational).

In an embodiment, the mechanical structure extends through at least one of the housing walls of the housing. Further, the mechanical structure, which extends through the at least one housing wall, may further comprise at least a portion of the moving mechanism, in particular at least one actuator, which is arranged outside of the housing and configured to move at least a portion of the mechanical structure for moving the optical component within the interior of the housing. Positioning the moving mechanism outside of the housing creates the advantage that less parts are arranged in the interior, which could negatively affect the optical component or the atmospheric environment in the interior of the housing e.g. by outgassing. The actuators may comprise cables, lubricated parts etc., which could create gasses in the interior, which could negatively affect the optical component. In addition, maintaining the moving mechanism is simpler, easier and more cost effective, when the moving mechanism is positioned at least partially outside of the housing in particular because the accessibility is higher. The mechanical structure, e.g. a rod, which extends through the housing wall, comprises a sealing, which seals between the housing wall and the mechanical structure in order to keep the atmospheric environment within the interior of the housing sealed from the exterior.

In an embodiment, the temperature control assembly comprises a heating cartridge, which is configured to provide heat to the optical component by using electrical energy. The heating cartridge is arranged for example at least partially arranged in the interior of the housing and contacts the optical component directly or indirectly for transferring the heat to the optical component. Direct contact means that no additional element is arranged between the heating cartridge and the optical component. Indirectly contacting means that at least one additional element is arranged between the heating cartridge and the optical component. The at least one additional element is for example a connecting element between the heating cartridge and the optical component, which has in particular relative high heat conducting properties e.g. the additional element is made of a metal in particular copper or silver.

In an embodiment, the temperature control assembly comprises a fluid circuit, which is configured to provide heat to and/or to remove heat from the optical component by circulating a fluid through the fluid circuit. The fluid circuit comprises fluid pipes, which extend to the optical component, in particular from the exterior of the housing to the optical components, and which are in thermal contact with the optical component in order to transfer heat from the fluid flowing through the pipes to the optical component or to transfer heat from the optical component to the fluid flowing through the pipes. The fluid circuit further may comprise a pump, a heat/cooling source for heating / cooling of the fluid and a control unit, which is configured to control the fluid circuit.

In an embodiment, the temperature control assembly comprises an induction heater, which is configured to provide heat to the optical component by using electromagnetic energy. The induction heater may comprise a coil, which is arranged in or at least one housing wall. The induction heater may further comprise an element, which interacts with the coil and which is suitable for induction heating. The element may be arranged at or near the optical component. The element is preferably arranged in the interior of the housing and thermally connected to the optical component for transferring the heat to the optical component. According to this embodiment, no cable duct or an opening in the housing for feeding a cable trough the housing is required. The induction heater is for example arranged on a plurality or all of the housing walls such that the optical component is heatable from a plurality of sides.

In an embodiment, the temperature control assembly comprises a thermal block, which is configured to provide heat to and/or to remove heat from the optical component by conducting heat through the thermal block to the optical component. The thermal block may comprise an electric resistance element arranged on one side of the thermal block, which is configured to convert electric current into heat, which is conducted to the other side of the thermal block arranged close to or at the optical component. The thermal block may be arranged on the optical component or may extend to the optical component to transfer heat to the optical component. The thermal block may be connected to an electric circuit, which controls the thermal transfer by regulating the electric current flowing through the thermal block. In particular through the electric resistance element. The thermal block is preferably arranged in the interior or may extend through the housing wall. In case the thermal block is arranged in the interior, the electric circuit may extend through the housing wall e.g. via a pin connection.

In an embodiment, the support assembly and the temperature control assembly form a single or combined assembly, which is configured to position the optical component in the interior of the housing and to control the temperature of the optical component. For example, the support assembly, which is configured to position the optical component within the interior of the housing may at least partially also be configured to control the temperature of the optical component e.g. in that cables, conduits etc. are guided through the support assembly. Alternatively or additionally, some portions of the support assembly may conduct heat to the optical component, such that the temperature control assembly is at least partially formed by the support assembly. In a simple embodiment, the support assembly and the temperature control assembly comprise only a heating element, e.g. a heat pipe formed as a rod, or a heating cartridge etc., which extend from at least one housing wall to the desired position of the optical component. The optical component is arranged at the tip portion of this support assembly such that it is positioned as desired. Further, the support assembly is configured to control the temperature of the optical component in that heat is transferred or removed from the optical component, in particular via rod, or a heating cartridge.

In an embodiment, the support assembly is at least partially hollow, thereby forming a cavity, which is closed towards the interior of the housing and open towards the exterior of the housing, and wherein the temperature control assembly is arranged at least partially in the cavity of the support assembly. The support assembly forms according to this embodiment a portion of the housing, namely the portion, which extends towards the optical component. The support assembly may be a hollow rod or hollow shaft, which is connected to the housing wall and which extends through an opening of the housing wall into the housing. The cavity formed by the support assembly is fluidically and/or atmospherically connected to the surrounding environment of the housing. The other side of the support assembly, in particular the other side of the hollow rod or shaft, is fluidically or atmospherically connected to the interior of the housing. In other words, the support assembly forms partially the housing. By positioning the temperature control assembly at least partially in the cavity of the support assembly, it is possible to advantageously heat the optical component arranged at the tip portion of the support assembly without the need of positioning the temperature control assembly in the interior of the housing. The cavity offers the possibility to position the temperature control assembly, e.g. the heating cartridge close to the optical component and at the same time to keep the temperature control assembly completely out of the interior of the housing. The heat is e.g. transferred via heat conduction or induction through the tip portion of the support assembly to the optical component.

In an embodiment, the ophthalmological optical device comprises a heat transfer element, which is arranged between the temperature control assembly and the optical component and which is configured to transfer heat from the optical component to the temperature control assembly and / or for transferring heat from the temperature control assembly to the optical component. The heat transfer element may further at least partially surround the optical component. At least partially surrounding may be determined in that the optical component is at least contacted from two spatial directions. The heat transfer element may comprise a plurality of components, which at least partially surround the optical component and are connected together such that the optical component is rigidly held by these components. The heat transfer element is the connecting element from the support assembly to the optical component, which thereby positions and holds the optical component and at the same time is configured to transfer heat from and to the optical component. The heat transfer element is e.g. made of a material having a relative high thermal conductivity of e.g. 50 W/(mK) or higher. The heat transfer element is e.g. made of copper or a copper alloy, aluminum or aluminum alloy, silver or silver alloy or a combination thereof.

The heat transfer element may further comprise components of the temperature control assembly, for transferring heat from and to the optical component. The components of the temperature control assembly are e.g. heating wires, a portion of the thermoblock, fluid pipes of the fluid circuit and / or a portion of the induction heater, which is configured to transfer the induced eddy currents into heat.

In an embodiment, the heat transfer element contacts the optical component at a plurality of surfaces of the optical component thereby partially or completely surrounding the optical crystal in at least one circumferential direction. The heat transfer to and from the optical component is advantageously uniformly, when the heat transfer element contacts as much surface of the optical component as possible. At least an input and output portion of the optical component for receiving and emitting the treatment laser beam needs to be free of the optical component, but the remaining surface could be contacted by the heat transfer element, thereby improving, in particular evenly distributing, the heat transfer from and to the optical component. The temperature control element is for example configured to provide heat to the optical component at a plurality of surfaces. E.g. a plurality of heating cartridges extend to a plurality of surfaces of the optical component.

In an embodiment, the ophthalmological optical device, in particular the temperature control assembly, comprises a temperature sensor, which is configured to provide a sensor signal, which is indicative of or characteristic for the temperature of the optical component. The temperature sensor is e.g. positioned in the interior of the housing, in particular at the vicinity of the optical component, or outside of the housing. In case the temperature sensor is positioned in the interior of the housing, the sensor signal is transferred trough the housing walls outside, in this case the sensor, in particular the respective element of the sensor, is advantageously positioned close to the optical component. In case the temperature sensor is positioned outside of the housing, the temperature of the optical component is for example measured indirectly via the temperature of the fluid flowing through the fluid circuit, or via other methods like infrared temperature measurements or pyrometers. In the latter case, the housing and where applicable, the heat transfer element, comprise openings towards the optical component for the infrared radiation of the temperature sensor. Further, at least one temperature sensor may be arranged in the heating cartridge or in the housing walls. In case the sensor is arranged in the cavity formed by the support assembly, the sensor signal is advantageously transferrable along the cavity outside of the cavity.

In an embodiment, the ophthalmological optical device, in particular the temperature control assembly, uses the sensor signal, which is provided by the temperature sensor, for controlling the temperature of the optical component. The sensor signal of the temperature sensor is e.g. used in a control loop or control algorithm implemented in the ophthalmological optical device as input for controlling the respective output, in particular of the temperature control assembly, for controlling the temperature of the optical component during the operation of the ophthalmological optical device.

In an embodiment, the optical device further comprises a control unit, which is configured to control at least the temperature control assembly for controlling the temperature of the optical component and / or which is configured to control the support assembly for positioning the optical component in the interior of the housing. The control unit may comprise a plurality of sub-units, which may perform different tasks, one may control the support assembly, in particular the moving mechanism, and another one may control the temperature control assembly. The control unit may be arranged in or at the ophthalmological optical device or in another device, which comprises the ophthalmological optical device or even further away. The control unit is configured to control the ophthalmological optical devices using sensor data and or control data or operation data, in particular data of the treatment laser beam, and / or other surrounding devices. The control unit enables to control the ophthalmological optical device reliably and as fast as desired. The control unit for controlling the ophthalmological optical device may be embedded in a control unit of the device in which the ophthalmological optical device is installed.

In an embodiment, the control unit is configured to control the temperature control assembly and / or the support assembly, in particular its moving mechanism, by using the at least one sensor signal, received, by the control unit from the at least one temperature sensor. An important task of the control unit is to control the temperature of the optical component during the operation of the ophthalmological optical device, such that the modification of the treatment laser beam remains as desired throughout the operation of the ophthalmological optical device, in particular remains unaffected by temperature changes of the surrounding environment and or by heat induction into the optical component during the operation. The sensor signal of the at least one or plurality of temperature sensors provides a highly valuable information for keeping the optical component within the desired temperature range. The control unit may use a control loop, in particular a closed-loop control loop, to control the temperature control assembly with the sensor signal of the temperature sensor as input.

In a further embodiment, the control unit is configured to control the temperature control assembly and / or the support assembly, in particular its moving mechanism, additionally or alternatively by using process parameter of the optical device, which affects the temperature of the optical crystal. During the operation of the ophthalmological device, the treatment laser beam can heat the optical component during its interaction with the optical component. The amount of heat and the heating duration is determined in particular by process parameters of the treatment laser beam, like wavelength, energy (pulse energy), energy density, pulse time, repetition rate, coherence length, polarization, beam dimensions, divergences etc. Further process parameter may include type of the optical component, ambient temperature, data from heat sinks or heat sources surrounding the ophthalmological optical device, operating profile of the treatment laser beam. Most of these process parameters are known in advance of the operation of the ophthalmological device and / or are measureable and provided to the control unit. The control unit can use these and / or other parameters to control the temperature control assembly and / or the support assembly.

In an embodiment, the housing comprises an inlet housing portion, which is at least partially transparent for the laser beam enabling the laser beam to penetrate into the interior of the housing and interacting with the optical component and/or an outlet housing portion, which is at least partially transparent for the laser beam enabling the laser beam to exit the interior of the housing. It might be conceivable that the inlet housing portion and the outlet housing portion are a single portion of the housing. In other words, the treatment laser beam penetrates into and out of the housing through the same transparent housing portion. The transparent portion is for example made of a crystal material, a glass material or plastic material. The material may comprise calcium-fluoride, quartz, magnesium-fluoride, sapphire or diamante material.

In an embodiment, the support assembly is arranged via a flange element at the housing, wherein the flange element is configured to seal between the support assembly and the housing. The flange element or flange portion offers a particular simple and robust solution to rigidly connect the support assembly to the housing.

In an embodiment, the ophthalmological optical device comprises in a single housing a plurality of optical components, which are arranged in parallel or in series with respect to the laser beam, wherein the plurality of optical components are arranged at one common support assembly or are arranged at one respective support assembly. According to this embodiment, the ophthalmological optical device comprises a plurality of optical components arranged in the interior of the single shared housing. The optical components are e.g. positioned in series, which means that a single treatment laser beam interacts with the optical components sequentially. In case the optical components are arranged parallel, each optical component might interact with a different treatment laser beam. In this embodiment, it is possible to create and control an optimized and controlled environment for a plurality of e.g. different optical components.

In an embodiment, the ophthalmological optical device comprises a plurality of housings arranged in parallel or in series, in which at least one optical component is arranged by at least one support assembly. The plurality of housings comprising e.g. each at least one support assembly and at least one temperature control assembly, which are configured to position the optical component and to control the temperature of the optical component. The ophthalmological device may comprise a single control unit, which is configured to control all of the support assemblies and all of the temperature control assemblies.

In an embodiment, support assembly extends from a lateral wall of the housing into the interior perpendicular or parallel with respect to the gravitational direction.

In a further embodiment, the ophthalmological optical device may comprise a pressure-setting pump, which is configured to control the gas pressure and / or the gas composition in the interior of the housing. The pressure-setting pump is in particular configured to create a negative pressure or vacuum in the interior and / or to insert and keep protective gas in the interior of the housing for enabling the desired optimized conditions in the interior of the housing during the operation of the ophthalmological optical device. The pressures setting pump is e.g. controlled by the control unit.

According to a further aspect, an ophthalmological laser treatment device is specified, which comprises the ophthalmological optical device as described above and hereinafter. The ophthalmological laser treatment device may further comprise additional comprising elements and modules necessary for performing the desired laser treatment of the eye of the patient e.g. a base station, a laser source, which generates the treatment laser beam, a power supply and other auxiliary subsystems necessary for operation of the ophthalmological laser treatment device.

The laser source is configured to, for example, generate an ultraviolet laser beam T having a wavelength of between 190 nm and 360 nm. For example, the laser source comprises a solid-state laser which produces such an ultraviolet laser beam T. The excimer laser uses a combination of a noble gas and a reactive gas under high pressure and electrical stimulation to generate the laser beam T. In particular, an excimer laser using argon as the noble gas and fluoride as the reaction gas may be used as the treatment laser source. The generated laser beam T may be, after its generation, directed or guided to the ophthalmological optical device as input treatment laser beam.

In another example, the laser source is configured to generate an infrared laser beam T having a wavelength of between 750 nm and 2000 nm. For example, the treatment laser source comprises a solid-state laser, such as a Ti:Sa, Erbium, Yterbium, Thulium, Holium and/or Neodym laser. In an embodiment, the treatment laser beam T is a pulsed laser beam, e.g. femtosecond laser pulses, which have pulse widths of typically from 10 fs to 1000 fs (1 fs = 10-15 s). The generated laser beam T may be, after its generation, directed or guided to the ophthalmological optical device as input treatment laser beam.

The ophthalmological laser treatment device may further comprise a laser scanner, which is configured to steer the treatment laser beam T delivered by the treatment laser source onto treatment points on a treatment pattern (comprising a laser trajectory). The ophthalmological laser treatment device may comprise an arm, which delivers the treatment laser beam to an application head through which the treatment laser beam leaves the ophthalmological laser treatment device onto the eye of the patient.

According to a further aspect of the present disclosure, a method for operating the ophthalmological optical device is specified.

The method comprises as a first step to provide an ophthalmological optical device as described above and / or hereinafter.

The method further comprises as a second step to modify at least one parameter of the treatment laser beam by interacting of the treatment laser beam with the optical component.

The method may further comprise as a third step to control, via the support assembly, the position of the optical component and / or to control via the temperature control assembly the temperature of the optical component.

The method may further comprise additional steps, as described above and hereinafter with respect to the ophthalmological optical device.

According to a further aspect of the present disclosure, the ophthalmological optical device may be used for applications outside of the ophthalmology. In this case, the device may be regarded as optical device, having all of the features and embodiments as described above and hereinafter. It is to be noted that the optical device for other application should thus be considered a separate inventive concept, which may be made subject of one or several divisional patent applications.

It is to be understood that both the foregoing general description and the following detailed description present embodiments, and are intended to provide an overview or framework for understanding the nature and character of the disclosure. The accompanying drawings are included to provide a further understanding, and are incorporated into and constitute a part of this specification. The drawings illustrate various embodiments, and together with the description serve to explain the principles and operation of the concepts disclosed.

### BRIEF DESCRIPTION OF THE DRAWINGS

The herein described disclosure will be more fully understood from the detailed description given herein below and the accompanying drawings, which should not be considered limiting to the disclosure described in the appended claims. The drawings are showing:
- **Fig. 1**: a first perspective view of an ophthalmological optical device according to a first exemplary embodiment;
- **Fig. 2**: a second perspective view of the ophthalmological optical device as shown in Figure 1, in which a front housing wall is suppressed, thereby enabling to see the interior of the housing;
- **Fig. 3**: a third perspective view of an ophthalmological optical device according to a second exemplary embodiment;
- **Fig. 4**: a fourth perspective view of an ophthalmological optical device according to a third exemplary embodiment;
- **Fig. 5**: a fifth perspective view of an ophthalmological optical device according to a fourth exemplary embodiment;
- **Fig. 6**: a sixth perspective view of an ophthalmological optical device according to a fourth exemplary embodiment.

### DETAILED DESCRIPTION OF THE DRAWINGS

Reference will now be made in detail to certain embodiments, examples of which are illustrated in the accompanying drawings, in which some, but not all features are shown. Indeed, embodiments disclosed herein may be embodied in many different forms and should not be construed as limited to the embodiments set forth herein; rather, these embodiments are provided so that this disclosure will satisfy applicable legal requirements. Whenever possible, like reference numbers will be used to refer to like components or parts.

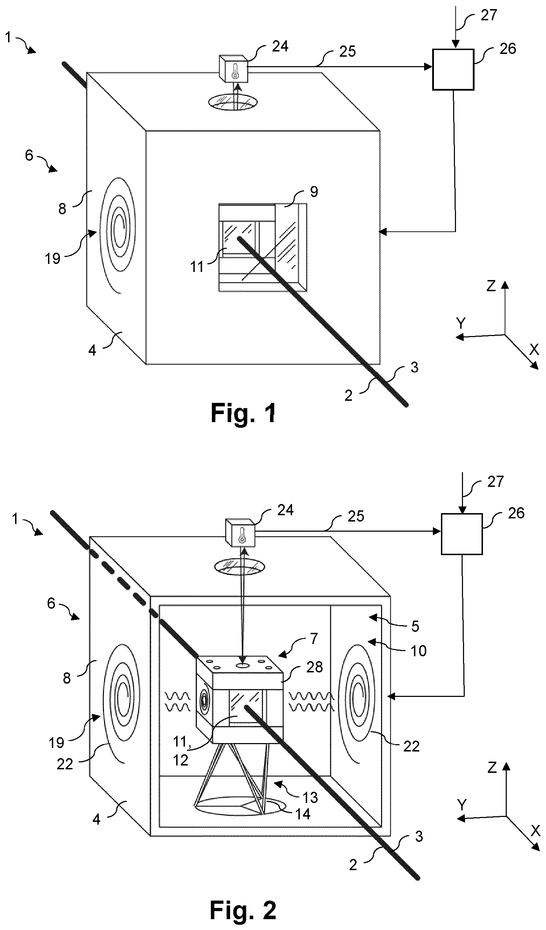

**Figure 1** shows an ophthalmological optical device 1 in a perspective view. The ophthalmological optical device 1 is configured to modify at least one parameter 3 of a treatment laser beam 2. The treatment laser beam 2 is configured to treat an eye of a patient during an ophthalmological procedure. The ophthalmological optical device 1 comprises a housing 4, determining an interior 5, which is surrounded or enclosed by the housing 4. The housing 4 further determines an exterior 6 of the housing 4. The exterior 6 is the space outside of the housing 4. The housing 4 has in this embodiment a cubic shape and has six housing walls 8. In other embodiments, the housing 4 may have a different shape, e.g. a cuboid shape, a cylindrical shape, a circular cylindrical shape or any further shape, which surrounds the interior 5. The housing walls 8 are made of metal, e.g. metal plates, which are welded together to form the housing 4. The housing 4 may alternatively be at least partially milled from a block. Figure 1 further advantageously shows an opening 9 in the housing wall 8, through which the treatment laser beam 2 penetrates as input laser beam into the interior 5 of the housing 4. The opening 9 is e.g. a recess or cut out in at least one of the housing walls 8. The opening 9 is covered with a laser transparent element enabling that the treatment laser beam 2 penetrates into the housing 4 and at the same time to fluidically seal between the interior 5 and the exterior 6 of the housing 4. The housing 4 is configured to provide in the interior 5 an atmospheric environment 10 (shown in Figure 2), which differs from a surrounding atmospheric environment. In other words, the housing 4 enables that in the interior 5 may prevail other atmospheric conditions compared to the atmospheric conditions exterior of the housing 4. The gas pressure and / or the atmospheric composition may vary in the interior 5 of the housing 4. The housing 4 forms in particular a hermetically sealed atmospheric environment in its interior. Further, the ophthalmological optical device 1 may comprise a pressure setting pump, which is configured to control the atmospheric environment in the interior 5 of the housing, e.g. by forming a negative pressure / vacuum in the interior 5 and / or by filling the interior with a protective gas.

Figure 1 further indicates through the transparent opening 9 an optical component 11 arranged in the interior 5 of the housing 4. **Figure 2** advantageously shows the interior 5 of the housing 4 including the optical component 11. The optical component 11 is arranged in the expected trajectory of the treatment laser beam 2 and is configured to modify at least one parameter 3 of the treatment laser beam 2 when the treatment laser beam 2 interacts with the optical component 11. Modifying at least one parameter 3 may include frequency conversions (e.g. frequency doubling) and / or parametric amplification and / or electro-optic modulation. Interacting with the optical component 11 may comprise that the treatment laser beam 2 is deflected by the optical component 11 and / or that the treatment laser beam 2 is guided through or is radiated through the optical component 11. The optical component 11 is e.g. an optical crystal 12, in particular a nonlinear crystal, which is made of Lithium Niobate (LiNbO3) and Tantalate (LiTaO3), Potassium niobate (KNbO3), Potassium titanyl phosphate (KTP, KTiOPO4) KTA (KTiOAsO4), RTP (RbTiOPO4) and RTA (RbTiAsPO4), Borate Crystals, Mid-IR Crystals.

**Figure 2** further advantageously shows the support assembly 13, which is arranged in the interior 5 of the housing 4 and which is configured to position the optical component 11 in the interior 5 of the housing 4. The support assembly 13 comprises according to this embodiment a mechanical structure 14, which extends from the bottom housing wall 8 to the optical component 11 for positioning the optical component 11 at the desired position in the interior 5. The mechanical structure 14 comprises a plurality of rods, which are connected to the housing wall 8 and to the optical component 11. It is also conceivable that the support assembly 13 extends from a plurality of housing walls 8 to the optical component 11. Further, the support assembly 13 may alternatively comprise a rope structure. The Figures, in particular Figure 2, further show that a heat transfer element 28 is arranged between the optical component 11 and the support assembly 13. The heat transfer element 28 is configured surround and hold the optical component 11 and to serve as a connecting element between the support assembly 13 and the optical component 11. The heat transfer element 28 may comprise a plurality of different elements, in particular made of a material having a high thermal conductivity (e.g. 50 W/(mK) or higher or 300 W/(mK) or higher), like aluminum, copper or silver. The different parts or elements of the heat transfer element 28 are e.g. screwed or welded together and surround the optical component 11 circumferentially in one direction, thereby enabling that the heat is advantageously transferred to or removed from as many sides as possible of the optical component 11. In other words, as much surface area as possible of the optical component 11 is contacted by the heat transfer element 28 for an improved and uniform heat transfer.

Figure 1 and Figure 2 further shows a temperature control assembly 19, which is configured to control the temperature of the optical component 11. The temperature control assembly 19 according to the Figures 1 and 2 comprises an induction heater 22. A first portion of the induction heater 22 is arranged in the housing walls 8, in particular the at least one coil of the induction heater 22. Further, a second portion of the induction heater 22 is arranged in the heat transfer element 28. The second portion is configured to transform the electromagnetic waves of the coil to heat energy. The heat transfer element 28 than further transfers the heat to the optical component 11. The temperature control assembly 19 comprises a first induction heater 22 arranged at one lateral side and a second induction heater 22 arranged at a second lateral side at the diametrically opposing position of the housing wall 8.

Figure 1 and Figure 2 further advantageously show a temperature sensor 24, which is configured to provide a sensor signal 25, which is characteristic or indicative of the temperature of the optical component 11. The temperatures sensor 24 is according to this embodiment arranged outside of the housing 4 and is configured to measure the temperature via infrared measurement through a respective transparent opening in the housing wall, which enables that the infrared waves of the sensor reach the optical component 11. The opening is covered with a respective transparent material. Figure 1 and Figure 2 further show schematically that the sensor signal 25 is provided to a control unit 26, which is configured to control the ophthalmological optical device 1. The ophthalmological optical device 1 may further comprise additional sensors, which are configured to provide further temperature data, position data and / or additional data, which may be used to control the ophthalmological optical device 1. It is also conceivable that the sensor signal 25 of the temperature sensor 24 is used by the ophthalmological optical device 1, in particular the temperature control assembly 19, for controlling the temperature of the optical component 11. In this case, the ophthalmological optical device 1 and / or the temperature control assembly 19 may provide directly the same functionality as the control unit 26 described above.

Figure 1 and Figure 2 further schematically show that process parameters 27 may be additionally used as input data for the control unit 26.

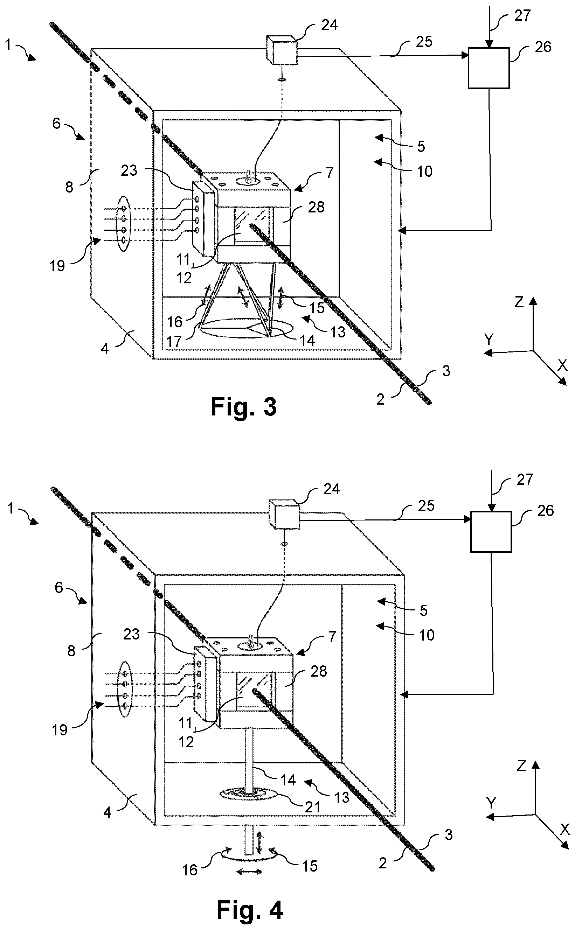

**Figure 3** shows in a perspective view a second embodiment of the ophthalmological optical device 1. This embodiment differs from the first embodiment shown with respect to Figure 1 and 2 in several details. The support assembly 13 comprises a moving mechanism 15, in particular six actuators 16, which is configured to move / newly position the optical component 11 within the interior 5 of the housing 4. The six actuators 16 form according to this embodiment a hexapod 17, which enables a particular free movement of the optical component 11.

Further, the embodiment of Figure 3 shows a different kind of temperature sensor 24. According to this embodiment is at least a portion of the temperature sensor 24, in particular its probe, is arranged in the interior 5 close or at the optical component 11. A cable is guided through the housing wall 8 in order to provide the sensor signal 25 to the control unit 26. The temperature sensor 24 may be a negative temperature coefficient sensor preferably arranged in a stainless steel sleeve. A wireless transfer would also be conceivable. Further, the temperature control assembly 19 comprises a thermal block 23, which is arranged on the heat transfer element 28. The thermal block 28 is connected via electric cables to an electric circuit, which provides electrical current to the thermal block 28, which is transferred to heat by the thermal block 28. The electric cables are guided through the housing wall 8, e.g. via a pin interface. The thermal block 28, in particular the electric circuit is controlled by the control unit 26, in particular in dependence of the sensor signal 25 of the temperature sensor 24 and / or in dependence of the process parameter 27. Similarly, the moving mechanism 15 may be controlled, by the control unit 26 in dependence of the sensor signal 25 and the process parameter 27, and / or an additional position signal, which is indicative of the position of the optical component 11 with respect to the beam path of the treatment laser beam 2 or the expected beam path of the treatment laser beam 2 or the housing 4. The thermal block 23 may further comprise a separate temperature sensor, which is configured to provide temperature signal indicate of the temperature of the thermal block 23, which is used as a control signal for the thermal block 23. In case this control signal fulfills a predetermined threshold, the electric circuit may be controlled respectively. In another embodiment, the temperature signal 25 of the temperature sensor 24 may further be used as a control signal for the temperature control assembly 19, in particular of the thermal block 23.

**Figure 4** shows a perspective view of an ophthalmological optical device 1 according to a third embodiment. This embodiment differs from the second embodiment referenced with respect to Figure 3 in particular in the support assembly 13. The support assembly 13 as shown in Figure 4 comprises as mechanical structure 14 a rod or shaft, which extends through the bottom housing wall 8 of the housing 4. The housing wall 8 comprises preferably a sealing mechanism, which is arranged between the housing wall 8 and the shaft and configured to provide a fluid tight sealing. The longitudinal end of the shaft extending into the interior 5 is connected to the heat transfer element 28 and / or the optical component 11 for holding and positioning of the optical component 11 in the interior 5. Figure 4 shows further that the moving mechanism 15 is arranged outside of the housing 4, which increases the accessibility and avoids contamination of the interior 5 by the moving mechanism 15. The moving mechanism 15 may comprise a plurality of actuators 16, which move the rod as indicated in Figure 4. Figure 4 further shows a sealing 21 arranged between the rod and the lower housing wall 8. The sealing 21 seals the atmospheric environment 10 in the interior 5 from the exterior 6 and enables at the same time the desired rotational and translational movement.

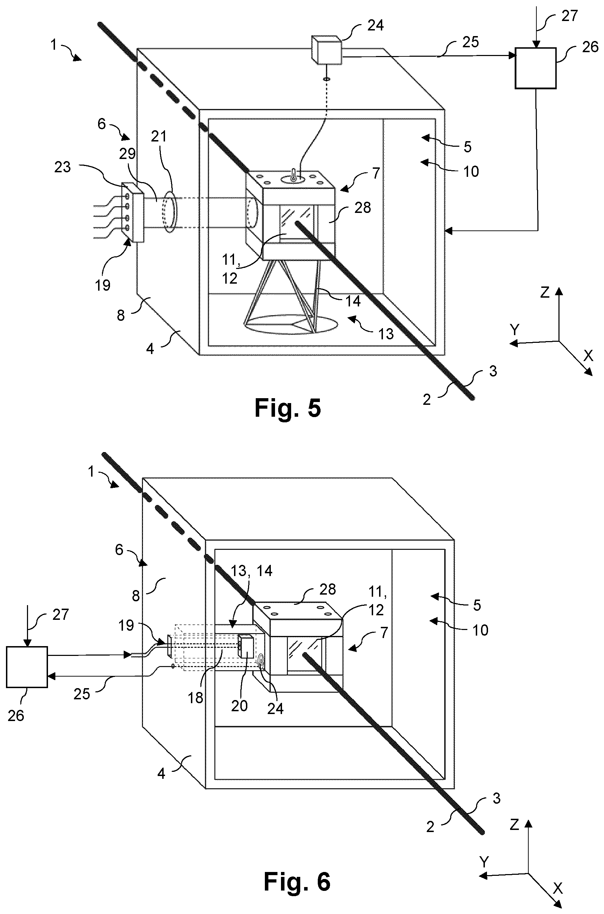

**Figure 5** shows a perspective view of an ophthalmological optical device according to a fourth embodiment. This embodiment differ from the previous described embodiment in particular in the temperature control assembly 19. The temperature control assembly 19 comprises according to this embodiment a thermal block 23 as described with respect to Figure 3 and 4. The difference is that the thermal block 23 is arranged outside of the housing 4. No cables need to be guided through the housing wall 8, which could potentially outgas in the interior 5 and thereby contaminate the interior 5. The thermal block 23 is thermally connected to a heat pipe 29, which is e.g. a metallic rod (copper rod) and which extends through the housing wall 8 to the heat transfer element 28 or directly to the optical component 11. The heat pipe 29 is configured to transfer the heat from the thermal block 23 to the optical component 11 or vice versa. A plurality of heat pipes 29 are of course also conceivable. Figure 5 further shows a sealing 21 arranged between the lateral housing wall 8 and the heat pipe 29, which seals between the heat pipe 29 and the housing wall 8 thermally such that the heat transfer from the heat pipe 29 to the housing wall 8 is reduced. The sealing 21 further seals the atmospheric environment 10 in the interior 5 from the exterior 6.

According to a further embodiment indicated in Figure 5, the heat pipe may alternatively or additionally comprise a fluid circuit, through which a heating or cooling fluid may flow for heating or cooling of the optical component 11.

**Figure 6** shows in a perspective view a fifth embodiment of the ophthalmological optical device 1. This embodiment differs in particular with respect to the support assembly 13 and the temperature control assembly 19 from the previous described embodiments. The support assembly 13 and the temperature control assembly 19 are at least partially integrated in each other. The mechanical structure 14 of the support assembly 13 is a partially hollow rectangular rod, which is open at one longitudinal end and forms thereby a cavity 18. The rod is connected at one longitudinal end to a lateral housing wall 8 and the optical component 11 is indirectly connected via the heat transfer element 28 at the other longitudinal end of the rod. The rod is for example screwed or welded to the housing wall. Further, the cavity 18 within the rod is open towards the housing wall 8 and closed towards the optical component 11. In other words, the hollow rod forms partially the housing 4 separating the interior 5 from the exterior 6. The housing 4 further comprises an opening, which connects the cavity 18 with the exterior 6. Through the opening, it is possible to guide cables into the cavity. Further, the support assembly 14 may comprise a bore in which at least partially the temperature sensor 24 is arranged. The temperature sensor 24 may also be guided through the opening in the housing and the cavity 18 towards the optical component 11. The temperature control assembly 19 comprises a heating cartridge 20, which is arranged in the cavity 18 of the support assembly 14. The wires of the heating cartridge 20 are guided through the opening in the housing and the cavity 18. The heating cartridge 20 is controlled by the control unit 26 and configured to provide heat to the heat transfer element 28 or directly to the optical component 11. 5.

It is of course conceivable that different elements of the different embodiments are combined to form a new embodiment, without deviating from the scope of the present disclosure.

### LIST OF REFERENCE SYMBOLS

- 1: Ophthalmological optical device
- 2: Treatment laser beam
- 3: Parameter of the treatment laser beam
- 4: Housing
- 5: Interior of the housing
- 6: Exterior of the housing
- 7: Center area of the housing
- 8: Housing wall
- 9: Opening in housing wall
- 10: Atmospheric environment
- 11: Optical component
- 12: Optical crystal
- 13: Support assembly
- 14: Mechanical structure
- 15: Moving mechanism
- 16: Actuator
- 17: Hexapod
- 18: Cavity
- 19: Temperature control assembly
- 20: Heating cartridge
- 21: Sealing
- 22: Induction heater
- 23: Thermal block
- 24: Temperature sensor
- 25: Sensor signal
- 26: Control unit
- 27: Process parameter
- 28: Heat transfer element
- 29: Heat pipe

- X: First lateral direction
- Y: Second lateral direction
- Z: Vertical direction

## Claims

1. An ophthalmological optical device (1) for modifying at least one parameter (3) of a treatment laser beam (2) used for treating an eye of a patient, the ophthalmological optical device (1) comprising:
a. a housing (4), which is configured to provide in an interior (5) of the housing (4) an atmospheric environment (10), which differs from an atmospheric environment outside the housing (4),
b. an optical component (11), which is arranged in the interior (5) of the housing (4), and which is configured to modify at least one parameter (3) of the treatment laser beam (2) when the treatment laser beam (2) interacts with the optical component (11),
c. a support assembly (13), which is arranged at least partially in the interior (5) of the housing (4) and which is configured to position the optical component (11) in the interior (5) of the housing (4),
d. a temperature control assembly (19), which is configured to control the temperature of the optical component (11),
e. a temperature sensor (24), which is configured to provide a sensor signal (25), which is indicative of the temperature of the optical component (11).

2. The ophthalmological optical device (1) according to claim 1, wherein the optical component (11) comprises an optical crystal (12), which is configured to modify at least one parameter (3) of the treatment laser beam (2) when the treatment laser beam (2) interacts with the optical crystal (13).

3. The ophthalmological optical device (1) according to any one of the preceding claims, wherein the optical component (11) is arranged in a center area (7) of the interior (5) of the housing (4), which is spaced apart from each housing wall (8) of the housing (4).

4. The ophthalmological optical device (1) according to any one of the preceding claims, wherein the support assembly (13) comprises a mechanical structure (14), which extends from at least one of the housing walls (8) to the optical component (11).

5. The ophthalmological optical device (1) according to any one of the preceding claims, wherein the support assembly (13) comprises a moving mechanism (15), which is configured to move the optical component (11) within the interior (5) of the housing (4).

6. The ophthalmological optical device (1) according to claim 5, wherein the moving mechanism (15) comprises:
a. at least one actuator (16), preferably a plurality of actuators (16), which is/are configured to move the optical component (11) within the interior (5) of the housing (4), and / or
b. at least one actuator (16), which is arranged outside of the housing (4) and configured to move at least a portion of the support assembly (13) arranged outside of the housing (4) for moving the optical component (11) within the interior (5) of the housing (4).

7. The ophthalmological optical device (1) according to any one of the preceding claims, wherein the temperature control assembly (19) comprises at least one of:
a. a heating cartridge (20), which is configured to provide heat to the optical component (11) by using electrical energy,
b. a fluid circuit , which is configured to provide heat to and/or to remove heat from the optical component (11) by circulating a fluid through the fluid circuit,
c. an induction heater (22), which is configured to provide heat to the optical component (11) by using electromagnetic energy, or
d. a thermal block (23), which is configured to provide heat to and/or to remove heat from the optical component (11) by conducting heat directly to the optical component (11) or indirectly via a heat pipe (29) to the optical component (11).

8. The ophthalmological optical device (1) according to any one of the preceding claims, wherein the support assembly (13) is at least partially hollow, thereby forming a cavity (18), which is closed towards the interior (5) of the housing (4) and open towards the exterior (6) of the housing (4), and wherein the temperature control assembly (19) is arranged at least partially in the cavity (18) of the support assembly (13).

9. The ophthalmological optical device (1) according to any one of the preceding claims, wherein the ophthalmological optical device (1) comprises a heat transfer element (28), which is arranged between the temperature control assembly (19) and the optical component (11), and which is configured to transfer heat from the optical component (11) to the temperature control assembly (19) and / or to transfer heat from the temperature control assembly (19) to the optical component (11).

10. The ophthalmological optical device (1) according to any one of the preceding claims, wherein the temperature control assembly (19) comprises the temperature sensor (24), which is configured to provide the sensor signal (25), which is indicative of the temperature of the optical component (11).

11. The ophthalmological optical device (1) according to any one of the preceding claims, wherein the ophthalmological optical device (1) further comprises a control unit (26), which is configured to control at least the temperature control assembly (19) for controlling the temperature of the optical component (11) and / or which is configured to control the support assembly (13), in particular its moving mechanism (15), for positioning the optical component (11) in the interior (5) of the housing (4).

12. The ophthalmological optical device (1) according to claim 11, wherein the control unit (26) is configured to control the temperature control assembly (19) and / or the support assembly (13) by using at least one of:
a. the at least one sensor signal (25), received by the control unit (26) from the at least one temperature sensor (24), or
b. process parameter (27), which influence the temperature of the optical component (11) during the operation of the ophthalmological optical device (1).

13. The ophthalmological optical device (1) according to any one of the preceding claims, wherein the ophthalmological optical device (1) comprises in a single housing (4) a plurality of optical components (11), which are arranged in parallel or in series with respect to the treatment laser beam (2), wherein the plurality of optical components (11) are arranged at one common support assembly (13) or are each arranged at one support assembly (13).

14. The ophthalmological optical device (1) according to any one of the preceding claims, wherein the ophthalmological optical device (1) comprises a plurality of housings (4) arranged in parallel or in series, in which at least one optical component (11) is arranged by at least one support assembly (13).

15. An ophthalmological laser treatment device comprising the ophthalmological optical device (1) as claimed in any one of the preceding claims.
